# FASCICULE DE BREVET EUROPEEN

(11) **EP 0 568 401 B1**
(45) Date de publication et mention de la délivrance du brevet: **06.11.1996**
(21) Numéro de dépôt: 93400712.1
(22) Date de dépôt: 19.03.1993
(51) Int. Cl.: A61F 13/02

(54) **Pansement adhésif transparent**
Durchsichtiger Klebeverband
Transparent adhesive dressing

(30) Priorité: 29.04.1992 FR 9205250
(43) Date de publication de la demande: 03.11.1993
(73) Titulaire: Laboratoire Hydrex (SA), 69550 Amplepuis (FR)
(72) Inventeur: Carion, Jean-Pierre, F-59850 Nieppe (FR)
(74) Mandataire: Lachat, Salomé

(56) Documents cités:
- EP-A- 0 066 899
- EP-A- 0 161 865
- EP-A- 0 189 999
- EP-A- 0 401 949

## Description

La présente invention est relative à un pansement adhésif transparent en matière synthétique.

Il s'agit plus particulièrement de pansements appelés couramment "champs à inciser", utilisés lors d'interventions chirurgicales. Des pansements adhésifs transparents sont utilisés également pour protéger la peau dans certains cas de lésions, telles que des brûlures superficielles ou erythèmes.

Lors d'interventions chirurgicales notamment, un champ à inciser est appliqué pour y adhérer sur le site opératoire, savoir la région de peau du patient dans laquelle sera pratiquée une incision. L'incision est faite simultanément à travers le champ à inciser et la peau. Le champ à inciser est retiré après l'intervention.

L'utilisation de champs à inciser présente l'avantage d'accroître la précision d'incision, de faciliter le maniement de la peau environnant l'incision et de diminuer le risque d'infection.

Les champs à inciser sont couramment constitués d'un morceau de film souple, par exemple de polyuréthane, découpé à partir d'un rouleau. La face adhésive du champ à inciser est protégée par un support, savoir le plus souvent une feuille de papier qui est enlevée au moment de l'utilisation. Les deux bords latéraux du champ à inciser comportent une bande de préhension, par exemple en papier ou en matière plastique, facilitant la manipulation lors de la mise en place sur le site opératoire. Les bandes de préhension sont généralement assemblées avec le morceau de film souple constituant le champ à inciser, par l'intermédiaire d'un bandeau latéral dudit morceau de film souple sur lequel elles adhèrent. Elles peuvent permettre également de retirer après utilisation le champ à inciser.

Etant donné l'extrême finesse et souplesse du morceau de polyuréthane ou autre film souple, les champs à inciser connus présentent l'inconvénient suivant: lorsque le support ou feuille de protection est enlevé du champ à inciser avant son application sur le site opératoire, le film souple a tendance à se tordre et à se fripper, sa face adhésive à se coller sur elle-même et à créer des plis collés de façon à rendre le pansement impropre à l'usage. Les produits connus ne sont pas d'un maniement aisé et les déchets dus au collage de la face adhésive sur elle-même avant application sur le site, sont importants.

Pour palier à l'inconvénient décrit, il a été proposé par exemple un pansement dont toute la périphérie de la face non adhésive est pourvue d'une bande de protection par exemple en papier, qui constitue un cadre de pose. Ce dernier est destiné à conférer une certaine rigidité au pansement avant son application sur le site. Le mode d'utilisation du pansement est le suivant: Le morceau de film souple transparent, ensemble avec son cadre de pose disposé sur la face non adhésive sont enlevés du support ou feuille de protection; le pansement maintenu par son cadre est appliqué sur le site; le cadre de pose est retiré.

Cette proposition constitue une amélioration des pansements connus, au moins lorsqu'il s'agit de pansements de petites dimensions car la fenêtre de polyuréthane ou autre film souple maintenue par le cadre de pose est très petite et la tendance du film de polyuréthane à se tordre et se coller sur lui-même est restreinte. Cette technique n'est pas adaptée pour des champs à inciser qui sont de dimensions plus grandes, par exemple de 15 cm x 28 cm car la surface de film de polyuréthane est trop grande pour ne pas entraîner de risques de collage.

Un autre type de pansement à deux couches est proposé par le brevet EP-A-0189999, à savoir un fim destiné à venir au contact de la peau, protégé sur sa face adhésive par une feuille de protection en deux parties se chevauchant au centre de la surface du pansement. Pour appliquer le pansement, les deux parties de la feuille de protection sont soulevées jusqu'à une zone latérale restant attachée à une zone latérale correspondantes du pansement proprement dans le but de pouvoir maintenir plus facilement le pansement au dessus de l'endroit de pose et de mieux guider sa mise en place sans qu'il ne se vrille et colle sur lui-même. Après pose sur la peau, les feuilles de protection sont retirées complètement par arrachement des zones latérales délimitées par des prédécoupes. Cette technique utile pour des pansements de petites dimensions est totalement inadaptée pour des pansements de plus grandes dimensions car il est impossible de maintenir en l'air la surface utile du pansement proprement dit en tenant les extrémités des feuilles de protection soulevées jusqu'aux zones latérales correspondantes.

Le brevet EP-A-0066899 divulgue un pansement à trois couches, se composant d'une feuille de film souple venant au contact de la peau du patient, avec une face adhésive recouverte avant application par une feuille de protection, et, d'une feuille de film moins souple donnant une certaine rigidité au pansement pour faciliter sa pose. La feuille de film moins souple est retirée après application du pansement sur la peau du patient en saisissant une patte d'enlèvement fixée à un endroit quelconque sur la surface extérieure de ladite feuille de film moins souple. Mais en enlevant la feuille de film moins souple, l'on risque de soulever la feuille de film souple au contact de la peau en raison d'un phénomène de dépression impliquant un effet succion.

Pour faciliter la mise en place des champs à inciser et accroître la sûreté d'utilisation, il serait souhaitable de donner une plus grande rigidité non seulement à la périphérie du champ à inciser mais à toute sa surface alors que cette dernière est très fine et souple par nature.

La présente invention a par conséquent pour objet un pansement adhésif transparent en matière synthétique, notamment un champ à inciser, du type précédemment décrit dont la manipulation est beaucoup plus fiable afin d'éviter des déchets en grand nombre, d'accroître la sûreté et rapidité d'utilisation tout en maintenant un coût le plus bas possible.

Pour atteindre ces objectifs, la présente invention propose un pansement transparent en matière synthétique,
- qui est composé, avant application sur le site opératoire, de trois couches, savoir une feuille en film souple, en particulier en polyuréthane, à une face adhésive, un support ou feuille de protection recouvrant ladite face adhésive, et, une feuille de film moins souple, en particulier en polyéthylène, disposée sur la face non adhésive du film souple,
- dont les bords latéraux comportent chacun une bande de préhension assemblée avec la feuille de film souple sur lequel elle adhère,
- dont la surface utile comprise entre les bandes de préhension latérales est délimitée, au moins sur un côté latéral du pansement, par une prédécoupe traversant au moins deux des trois couches,
- dont au moins l'une des prédécoupes latérales est protégée par une bande de renfort, disposée sur la feuille de matériau moins souple, en particulier en polyéthylène.

Le pansement conforme à l'invention présente l'avantage d'une rigidité, avant application sur le site, suffisante pour permettre un maniement aisé. Tout risque que la partie en polyuréthane se torde, se frippe et se colle sur elle-même est ainsi évité.

Etant donné que les bandes continues de polyuréthane, à partir desquelles sont découpés les pansements ou champs à inciser pour confectionner des produits prêts à l'emploi, sont fabriquées avec une feuille protectrice en polyéthylène qui adhère sur la face non adhésive du pansement par phénomène statique, la préparation des pansements ou champs à inciser stériles, à usage unique, prêts à l'emploi, n'implique aucune étape de travail supplémentaire et n'occasionne aucun accroissement de coût.

Selon l'invention, la feuille de film souple est avantageusement en polyuréthane, la feuille de souplesse moindre en polyéthylène. Le polyuréthane qui est un matériau microporeux, est perméable à la vapeur d'eau et à l'oxygène mais empêche toute pénétration bactérienne de l'extérieur. Tout autre matériau équivalent peut être utilisé dans le cadre de la présente invention.

Des détails de réalisation d'un pansement conforme à l'invention et les procédés d'application correspondants sont précisés par les revendications 2 à 5.

L'invention sera décrite de façon purement indicative à l'aide des dessins annexés à titre d'exemple non limitatif.

La figure 1 présente une coupe d'un premier mode de réalisation d'un pansement adhésif conforme à l'invention, comportant deux prédécoupes latérales.

La figure 2 présente une coupe d'un second mode de réalisation d'un pansement adhésif conforme à l'invention, comportant une prédécoupe latérale.

La figure 3 présente une coupe partielle d'un troisième mode de réalisation d'un pansement adhésif conforme à l'invention, comportant une bande de renfort saillante.

L'on trouvera in fine une nomenclature des différents repères utilisés au cours de la description.

Un pansement, par exemple un champ à inciser, est constitué de trois couches:
- une feuille de film souple (1) en matière synthétique possédant une face adhésive (10) et une face non adhésive,
- un support ou feuille de protection (3) de la face adhésive (10),
- une feuille de film en matériau moins souple (2), disposée sur la face non adhésive de la feuille souple (1).

Dans le cadre de la présente invention, il est particulièrement avantageux de prévoir que la feuille de film souple (1) soit en polyuréthane et la feuille de film moins souple (2) en polyéthylène.

Le polyuréthane étant un matériau microporeux, il présente l'avantage de permettre l'évaporation de l'eau et d'éviter toute macération entre le champ à inciser et la peau, tout en empêchant la pénétration bactérienne de l'extérieur, ce qui diminue le risque d'infection.

De plus, comme la présence du film de polyéthylène est nécessaire lors de la fabrication des bandes continues enroulées de polyuréthane, à partir desquelles sont découpés et préparés les pansements stérils prêts à l'usage, le type de fabrication conforme à l'invention n'implique aucun accroissement de coût.

Mais dans le cadre de la présente invention, il est également possible que la feuille de film souple soit constituée d'un autre matériau répondant au même critère que le polyuréthane. Il en est de même de la feuille de matériau moins souple, destinée uniquement à donner au pansement une certaine rigidité avant et lors de l'application sur le site.

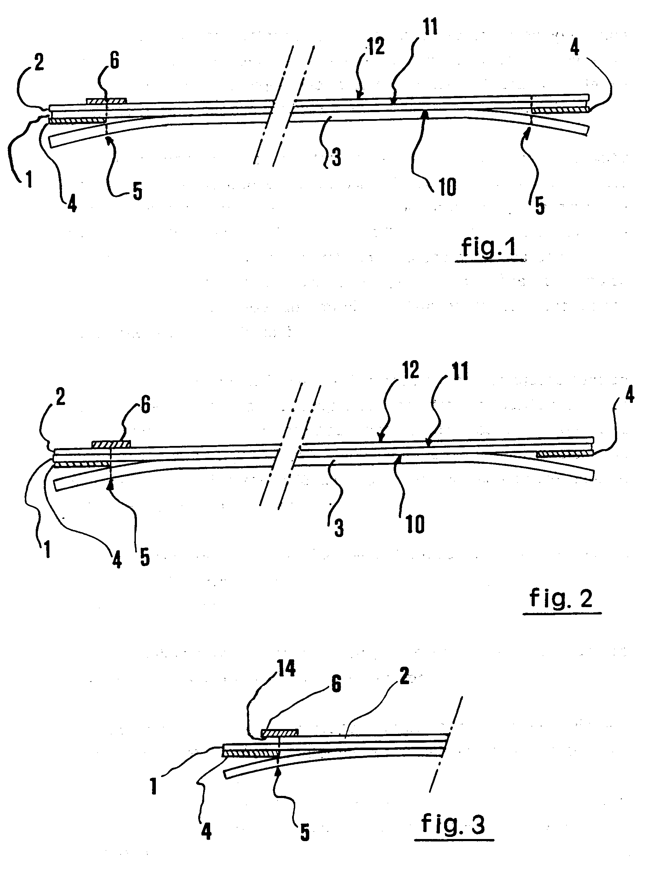

Selon un mode préférentiel de l'invention, montré à la figure 1, chacun des bords latéraux du pansement comporte une bande de préhension (4), par exemple en matière synthétique colorée, disposée entre la feuille de polyuréthane (1) et le support ou feuille de protection (2). La bande de préhension (4) est assemblée avec le film de polyuréthane (1) sur lequel elle adhère. La surface utile (12) du pansement est délimitée de part et d'autre par une prédécoupe (5) traversant les trois couches (1), (2), (3) du pansement. Sur l'une des découpes (5) est avantageusement disposée une bande de renfort (6) confortant l'assemblage avec le film de polyéthylène (2).

L'application du pansement conforme à l'invention est très aisée et d'une grande sécurité. La présence de la feuille de polyéthylène (2) donne au pansement proprement dit, savoir la feuille de polyuréthane (1) la rigidité nécessaire pour éviter que ce dernier ne se torde et se colle sur lui-même.

Le mode d'application du pansement conforme au mode de réalisation montré à la figure 1, est le suivant: l'on enlève d'abord le support ou feuille de protection (3) de la face adhésive (10) de la feuille de polyuréthane (1) qui reste à ce stade assemblée avec la feuille de polyéthylène (2) par phénomène statique. Le pansement est appliqué sur le site opératoire. L'on enlève la bande de préhension (4), par déchirure le long de la prédécoupe (5) non protégée par une bande de renfort (6). Puis, est ôtée la seconde bande de préhension (4) ensemble avec la feuille de polyéthylène (2) qui se sépare très facilement de la feuille de polyuréthane (1).

Selon un autre mode de réalisation de l'invention montré à la figure 2, il est également possible de prévoir une prédécoupe (5) traversant les trois couches (1), (2), (3) uniquement sur l'un des côtés latéraux du pansement. La prédécoupe comporte alors une bande de renfort (6).

L'application du pansement comportant une seule prédécoupe latérale (5) se fait comme suit: comme précédemment, l'on enlève d'abord le support ou feuille de protection (3) de la face adhésive (10) de la feuille de polyuréthane (1) protégée par une feuille de polyéthylène (2) sur la face non adhésive (11), qui y adhère par phénomène statique. Le pansement est appliqué sur le site opératoire. Puis, est enlevée, par déchirure le long de la prédécoupe unique (5) protégée par une bande de renfort, la bande de préhension (4) ensemble avec la feuille de polyéthylène (2). La bande de préhension (4) disposée du côté latéral sans prédécoupe reste attachée à la feuille de polyuréthane (1) appliquée sur le site opératoire. La bande de préhension qui reste sur place, facilitera l'enlèvement du pansement du site opératoire après l'intervention. Ce mode de réalisation est particulièrement adapté pour des pansements de grande dimension.

La figure 3 montre un mode de réalisation particulièrement avantageux d'un pansement adhésif conforme à l'invention. Le pansement est constitué comme décrit précédemment mais d'un côté latéral, la feuille de polyéthylène (2) ne s'étend que jusqu'à la prédécoupe (5) de sorte que la bande de renfort (6) est saillante sur ladite feuille de film moins souple (2). La surface inférieure (13) de la partie saillante (14) de la bande de renfort (6) n'est alors pas adhésive.

L'application du pansement conforme à la figure 3 se fait comme suit: comme précédemment, l'on enlève d'abord le support ou feuille de protection (3) de la face adhésive (10) de la feuille de polyuréthane (1) dont la face non adhésive est protégée par une feuille de polyéthylène (2) servant de raidisseur et assemblée avec la feuille (1) par phénomène statique. Ensuite, le pansement est appliqué sur le site opératoire. Puis, l'on enlève, par soulèvement de la bande de renfort saillante (6), le film protecteur de polyéthylène (2) en maintenant avantageusement de l'autre main la bande de préhension (4) disposée du même côté latéral que la bande de renfort (6), cette dernière servant également de bande d'enlèvement. Les bandes de préhension peuvent rester sur place pendant l'intervention. Elles faciliteront l'enlèvement du pansement du site opératoire après l'intervention.

## Revendications

1. Pansement adhésif transparent en matière synthétique composé, avant application, de trois couches (1), (2), (3), savoir une feuille de film souple, en particulier en polyuréthane (1), à une face adhésive (10), un support ou feuille de protection (3) recouvrant ladite face adhésive, et, une feuille de matériau moins souple, en particulier de polyéthylène (2), disposée sur la face non adhésive (11) du film souple (1), *caractérisé en ce que*
- chacun de ses bords latéraux comporte une bande de préhension (4) assemblée avec la feuille de film souple (1) sur lequel elle adhère,
- sa surface utile (12) comprise entre les bandes de préhension latérales (4) est délimitée, au moins sur un côté latéral du pansement, par une prédécoupe (5), traversant au moins les couches (1) et (2) des trois couches,
- dont au moins l'une des prédécoupes latérales (5) est protégée par une bande de renfort (6) disposée sur la feuille de film moins souple, en particulier en polyéthylène (2).

2. Procédé d'application d'un pansement adhésif transparent conforme à la revendication 1, qui comporte deux prédécoupes latérales (5), *caractérisé par* les étapes suivantes:
- enlèvement du support ou feuille de protection (3) de la face adhésive (10) de la feuille de film souple (1) assemblée avec la feuille de film moins souple (2),
- application du pansement sur le site opératoire,
- enlèvement, par déchirure le long de la prédécoupe (5) non protégée par une bande de renfort (6), de la bande de préhension (4),
- enlèvement de la seconde bande de préhension (4) ensemble avec la feuille de film moins souple (2).

3. Procédé d'application d'un pansement adhésif transparent conforme à la revendication 1, qui comporte une seule prédécoupe latérale (5), *caractérisé par* les étapes suivantes:
- enlèvement du support ou feuille de protection (3) de la face adhésive (10) de la feuille de film souple (1) assemblée avec la feuille de film moins souple (2),
- application du pansement sur le site opératoire,
- enlèvement, par déchirure le long de la prédécoupe (5) protégée par une bande de renfort (6), de la bande de préhension (4) ensemble avec la feuille de film moins souple (2), la bande de préhension (4) disposée du côté sans prédécoupe restant en place.

4. Pansement adhésif transparent en matière synthétique selon la revendication 1 *caractérisé en ce que* au moins d'un côté latéral, la feuille de film moins souple (2) ne s'étend que jusqu'à la prédécoupe (5) de sorte que la bande de renfort (6) est saillante sur ladite couche (2), la face inférieure (13) de la partie saillante (14) de la bande de renfort (6) n'étant pas adhésive.

5. Procédé d'application d'un pansement adhésif transparent conforme à la revendication 4, *caractérisé par* les étapes suivantes:
- enlèvement du support ou feuille de protection (3) de la face adhésive (10) de la feuille de film souple (1) assemblée avec la feuille de film moins souple (2),
- application du pansement sur le site opératoire,
- enlèvement de la feuille de film moins souple (2) par soulèvement de la bande de renfort (6).

## Claims

1. A transparent adhesive dressing of synthetic material constituted, prior to application, by three layers (1, 2, 3), namely a sheet of flexible film, in particular of polyurethane (1), having an adhesive face (10), a protective sheet or backing (3) covering said adhesive face, and a sheet of less flexible material, in particular polyethylene (2), disposed on the non-adhesive face (11) of the flexible film (1), the dressing being characterized in that:
each of its lateral edges includes a strip for grasping (4) assembled to the sheet of flexible film (1) to which it adheres;
its useful area (12) extending between the lateral strips for grasping (4) is delimited, at least on one lateral side of the dressing, by a starter cut (5) passing through at least two (1 and 2) of the three layers; and
at least one of the lateral starter cuts (5) is protected by a reinforcing strip (6) disposed on the sheet of less flexile film, in particular the sheet made of polyethylene (2).

2. A method of applying a transparent adhesive dressing according to claim 1, the dressing including two lateral starter cuts (5), the method being characterized by the following steps:
removing the protective sheet or backing (3) from the adhesive face (10) of the sheet of flexible film (1) which is assembled to the sheet of less flexible film (2);
applying the dressing to the site of the operation;
removing the strip for grasping (4) by tearing along the starter cut (5) that is not protected by a reinforcing strip (6); and
removing the second strip for grasping (4) together with the sheet of less flexible film (2).

3. A method of applying a transparent adhesive dressing according to claim 1, which dressing includes only one lateral starter cut (5), the method being characterized by the following steps:
removing the protective sheet or backing (3) from the adhesive face (10) of the sheet of flexible film (1) assembled to the sheet of less flexible film (2);
applying the dressing to the site of the operation; and
removing the strip for grasping (4) together with the sheet of less flexible film (2) by tearing along the starter cut (5) that is protected by a reinforcing strip (6), the strip for grasping (4) disposed on the side that does not include a starter cut remaining in place.

4. A transparent adhesive dressing of synthetic material according to claim 1, characterized in that at at least one lateral side the sheet of less flexible film (2) extends no further than the starter cut (5) such that the reinforcing strip (6) projects over said layer (2), the bottom face (13) of the projecting portion (14) of the reinforcing strip (6) not being adhesive.

5. A method of applying a transparent adhesive dressing according to claim 4, characterized by the following steps:
removing the protective sheet or backing (3) from the adhesive face (10) of the sheet of flexible film (1) assembled to the sheet of less flexible film (2);
applying the dressing to the site of the operation; and
removing the sheet of less flexible film (2) by lifting off the reinforcing strip (6).

## Patentansprüche

1. Durchsichtiger Klebeverband aus Kunststoff, bestehend vor Gebrauch aus drei Schichten (1), (2), (3), nämlich aus einer dünnen, biegsamen, einseitig eine Klebeoberfläche aufweisenden Folie (1), insbesondere aus Polyurethan, einer die Klebeoberfläche der biegsamen Folie (1) bedeckenden Stütz- oder Schutzfolie (3) und einer auf der nicht klebenden Oberfläche der biegsamen Folie (1) angeordneten Folie (2) aus weniger biegsamem Material, insbesondere aus Polyäthylen, dadurch gekennzeichnet, daß
- beide Seitenränder des Klebeverbandes je mit einem durch Hatten auf der biegsamen Folie (1) mit dem Klebeverband verbundenen Greifband (4) versehen sind,
- die sich zwischen den beiden seitlichen Greifbändern (4) erstreckende Nutzfläche (12) des Verbandes wenigstens einseitig durch eine Reißlinie (5) begrenzt ist, wobei die Perforation der Reißlinie wenigstens zwei (1), (2) der drei Verbandsschichten durchsetzt,
- wenigstens eine der seitlichen Reißlinien (5) durch ein auf der weniger biegsamen Folie (2) aus Polyäthylen angeordnetes Verstärkungsband (6) geschützt ist.

2. Applikationsverfahren für einen durchsichtigen Klebeverband nach Anspruch 1 mit zwei seitlichen Reißlinien (5), gekennzeichnet durch die folgenden Verfahrensschritte:
- Entfernen der Stütz- oder Schutzfolie (3) von der Klebeoberfläche (10) der mit der weniger biegsamen Folie (2) verbundenen Folie (1) aus dünnem, biegsamem Material,
- Applikation des Verbandes auf die Eingriffsstelle,
- Entfernen des Greifbands (4) durch Wegreissen entlang der nicht durch ein Verstärkungsband (6) geschützten Reißlinie (5),
- Entfernen des zweiten Greifbandes (4) zusammen mit der weniger biegsamen Folie (2).

3. Applikationsverfahren für einen durchsichtigen Klebeverband nach Anspruch 1 mit einer seitlichen Reißlinie (5), gekennzeichnet durch die folgenden Verfahrensschritte:
- Entfernen der Stütz- oder Schutzfolie (3) von der Klebeoberfläche (10) der mit der weniger biegsamen Folie (2) verbundenen Folie (1) aus dünnem, biegsamem Material,
- Applikation des Verbandes auf die Eingriffsstelle,
- Entfernen, durch Wegreissen entlang der durch ein Verstärkungsband (6) geschützten Reißlinie (5), des Greifbandes (4) zusammen mit der weniger biegsamen Folie (2), wobei das auf der keine Reißlinie aufweisenden Seite des Verbandes angeordente Greifband (4) an Ort und Stelle bleibt.

4. Durchsichtiger Klebeverband aus Kunststoff nach Anspruch 1, dadurch gekennzeichnet, daß sich die weniger biegsme Folie (2) wenigstens einseitig nur bis zur Reißlinie (5) erstreckt, so daß das Verstärkungsband (6) über die Folie (2) vorsteht, wobei die Unterseite (13) des vorstehenden Teils (14) des Verstärkungsbandes (6) nicht klebend ausgebildet ist.

5. Applikationsverfahren für einen durchsichtigen Klebeverband nach Anspruch 4, gekennzeichnet durch die folgenden Verfahrensschritte:
- Entfernen der Stütz- oder Schutzfolie (3) von der Klebeoberfläche (10) der mit der weniger biegsamen Folie (2) verbundenen Folie (1) aus dünnem, biegsamem Material,
- Applikation des Verbandes auf die Eingriffsstelle,
- Entfernen der weniger biegsamen Folie (2) durch Hochheben des Verstärkungsbandes (6).
